# EUROPEAN PATENT SPECIFICATION

(11) **EP 2 460 515 B1**
(45) Date of publication and mention of the grant of the patent: **08.03.2017**
(21) Application number: 12155823.3
(22) Date of filing: 07.08.2008
(51) Int. Cl.: A61K 9/20, A61K 31/198, A61K 38/23, A61K 9/16

(54) **Oral calcitonin compositions and applications thereof**
Orale Calcitonin-Zusammensetzungen und ihre Anwendungen
Compositions à base de calcitonine orale et leurs applications

(30) Priority: 09.08.2007 US 954874 P
(43) Date of publication of application: 06.06.2012
(62) Divisional of application: 08787006.9
(73) Proprietor: Novartis AG, 4056 Basel (CH)
(72) Inventor: Arnold, Michel, 68220 Wentzwiller (FR); Azria, Moise, 4054 Basel (CH)
(74) Representative: Fabry, Bernd

(56) References cited:
- WO-A1-92/21355
- WO-A1-2004/012772
- WO-A1-2005/014031
- CHESNUT CHARLES H III ET AL: "A randomized trial of nasal spray salmon calcitonin in postmenopausal women with established osteoporosis: The prevent recurrence of osteoporotic fractures study", AMERICAN JOURNAL OF MEDICINE, vol. 109, no. 4, September 2000 (2000-09), pages 267-276, XP002538084, ISSN: 0002-9343

## Description

### Field of the Invention

The present invention relates to compositions for the oral delivery of pharmacologically active calcitonin, to methods of synergistically enhancing the biological effects of orally administered calcitonin, and to methods of treating and/or preventing disease in mammals, particularly humans, by orally administering calcitonin compositions in accordance with the invention.

### Background of the Invention

The calcium metabolism in humans comprises a flow to and from the bone that is normally neutral, about 5 mmol is turned over per day. Skeletal bones serve as an important storage for calcium, containing about 99% of the total body calcium. Calcium is released from bone by parathyroid hormone, whereas calcitonin stimulates incorporation of calcium in bone. The regulatory organ for bone metabolism is the parathyroid that produce parathyroid hormone in response to low calcium levels. The parafollicular cells of the thyroid produce calcitonin in response to high calcium levels.

Oral delivery of pharmacologically active agents is generally the delivery route of choice since it is convenient, relatively easy and generally painless, resulting in greater patient compliance relative to other modes of delivery. However, biological, chemical and physical barriers such as varying pH in the gastrointestinal tract, powerful digestive enzymes, and active agent impermeable gastrointestinal membranes, makes oral delivery of some pharmacologically active agents to mammals problematic, e.g. the oral delivery of calcitonins, which are long-chain polypeptide hormones secreted by the parafollicular cells of the thyroid gland in mammals and by the ultimobranchial gland of birds and fish. It has proven difficult to administer calcitonin orally due to the insufficient stability of calcitonin in the gastrointestinal tract as well as the inability of calcitonin to be readily transported through the intestinal walls into the blood stream. U.S. Pat. Nos. 5,773,647 and 5,866,536 describe compositions for the oral delivery of active agents, such as heparin and calcitonin, with modified amino acids, such as, N-(5-chlorosalicyloyl)-8-aminocaprylic acid (5-CNAC), N-(10-[2-hydroxybenzoyl]aminodecanoic acid (SNAD), and N-(8-[2-hydroxybenzoyl]amino) caprylic acid (SNAC). However, relatively high amounts of calcitonin peptides are required for these oral compositions to achieve the desired biological effects.

### Summary of the invention

The present invention is directed to pharmaceutical compositions which enhance the biological effects of orally administered calcitonin and allow for a more convenient administration and a better compliance of patients to calcitonin therapy. The invention is particularly directed at compositions comprising one or more of the delivery agents 5-CNAC, SNAD and/or SNAC with calcitonin peptides.

Thus, the invention provides oral pharmaceutical compositions comprising an oral delivery agent selected from the group consisting of N-(5-chlorosalicyloyl)-8-aminocaprylic acid (5-CNAC), N-(10-[2-hydroxy benzoyl]amino)decanoic acid (SNAD), N-(8-[2-hydroxybenzoyl] amino)caprylic acid (SNAC), and a hydrate, solvate or a salt thereof; and from about 0.1 to 2.5 mg of calcitonin; and
a) a D type vitamin in an amount of from 200 to 1000 IU; and/or
b) a calcium salt in an amount of from 200 to 1000 mg free calcium

In a further embodiment, the invention is directed to a method for enhancing the effect of oral calcitonin, said method comprising administering to a subject in need of said pharmacologically active compositions an effective amount of a pharmaceutical composition according to the instant invention.

In a still further embodiment, the invention is directed to a method of treating or preventing bone related diseases and calcium disorders comprising administering to a patient an oral pharmaceutical composition of the present invention.

In a related embodiment, the present invention provides a method of preventing or treating disorders responsive to the action of calcitonin in a patient in need thereof comprising administering orally to said patient an oral pharmaceutical composition of the present invention.

The present invention also provides the use of a combination of calcitonin, a D-type vitamin and calcium for the manufacture of a medicament for preventing or treating disorders responsive to the action of calcitonin.

In a related embodiment, the present invention provides a combination of calcitonin, a D-type vitamin and calcium for preventing or treating disorders responsive to the action of calcitonin.

The present invention further provides a kit comprising:
a) an oral pharmaceutical composition comprising calcitonin;
b) an oral delivery agent selected from the group consisting of N-(5-chlorosalicyloyl)-8-aminocaprylic acid, N-(10-[2-hydroxybenzoyl]amino decanoic acid or N-(8-[2-hydroxybenzoyl]amino)caprylic acid, and a hydrate, solvate or salt thereof;
c) a composition comprising a D-type vitamin,
d) a composition comprising calcium, and optionally
e) written instructions which instructions provide that said oral pharmaceutical composition may be taken prior to the consumption of food.

Further features and advantages of the invention will become apparent from the following detailed description of the invention.

### Detailed description of the invention

### Definitions:

Calcitonin is effective in the treatment and prevention of osteoporosis and in reducing the bone pain associated with osteoporosis and some bone tumors. Calcitonin has been used in humans as an injectable for over 30 years. It has proven to be very safe. A known class of pharmacologically active agents, calcitonins have varying pharmaceutical utility and are commonly employed in the treatment of e.g. Paget's disease, hypercalcemia and postmenopausal osteoporosis. Various calcitonins, including salmon, pig and eel calcitonin are commercially available and commonly employed for the treatment of e.g. Paget's disease, hypercalcemia of malignancy and osteoporosis. The calcitonin can be any calcitonin, including natural, synthetic or recombinant sources thereof, as well as calcitonin derivatives such as 1,7-Asu-eel calcitonin. The compositions can comprise a single calcitonin or any combination of two or more calcitonins. The preferred calcitonin is synthetic or recombinant salmon calcitonin. The calcitonins are commercially available or may be synthesized by known methods.

As used herein, "calcitonin" refers to a class of pharmacological agents used for the treatment of, e.g., Paget's disease, hypercalcemia, osteoporosis, and disorders characterized by arthritic conditions. e.g. osteoarthritis, including natural, synthetic or recombinant human, salmon, pig or eel calcitonin. Calcitonin is a 32 amino acid polypeptide hormone that is produced in humans primarily by the Parafollicular (also known as C) cells of the thyroid, and in many other animals in the ultimobranchial body. Preferred for the invention is the use of salmon calcitonin (sCT). The calcitonins are commercially available or may be synthesized by known methods. Synthetic salmon calcitonins are, for example, available as Miacalcin™ and Calcimar™.

"Disorders responsive to the action of calcitonin" are, e.g., various types of bone disorders. A first class of disorders fall in the category relating to disorders caused by bone resorption. Examples of such disorders are hypercalcemia, osteoporosis, osteolyisis and Paget's disease, a chronic bone disorder that typically results in enlarged, deformed bones due to excessive breakdown and formation of bone tissue that can cause bones to weaken and may result in bone pain, arthritis, deformities or fractures. A second class of disorders is characterized by arthritic conditions. An example of such disorders is osteoarthritis. Rheumatoid arthritis (RA), a chronic, systemic, inflammatory autoimmune disease, has as its primary target the synovial tissues. RA is characterized by inflammation and swelling of skeletal joints, especially the small joints of the extremities, leading to erosion and destruction of cartilage and bone. The present invention may be used to inhibit, halt or even reverse the cartilage and bone erosion, fracture and destruction, and to decrease the pain, associated with rheumatoid arthritis.

The term "about" as used herein denotes both the actual numbers of values cited, as well as a range falling within up to 10% below and above the cited numbers or values.

A "calcitonin delivery agent" is a compound or composition which is useful in the delivery of calcitonin selected biological systems. Suitable oral delivery agents are, for example, those described in U.S. Patent Nos. 5,773,647 and 5,866,536, as well as International Application WO 00/59863, the contents of which are incorporated herein by reference. Specific embodiments thereof are 5-CNAC, SNAD and SNAC, and the salts and hydrates and solvates thereof, such as the ethanol solvates. The highly preferred disodium salts, monohydrates and ethanol solvates are described in International Application WO 00/59863, including their preparation.

The term "oral" as used herein comprises any kind of oral delivery routes (comprising buccal and sublingual routes).

"Delivery agent" as used herein refers to carrier compounds or carrier molecules that are useful in the oral delivery of therapeutic agents. "Delivery agent" may be used herein interchangeably with "carrier".

By a "therapeutically effective amount" of calcitonin as provided in the oral dosage forms according to the present invention is to be understood as an amount of calcitonin which is sufficient to achieve a clinically significant improvement of a condition associated with Rheumatoid arthritis in a human or animal patient such as, for example, inhibition of inflammation of the joints or the of the swelling of skeletal joints, inhibition, halting and/or reversion of cartilage and bone erosion and destruction, and/or decrease of pain or which is sufficient to prevent the onset of said conditions. For osteoporosis and osteoarthritis indications the therapeutically effective amount of calcitonin is understood to give clinically significant improvement in terms of reduced risk of fractures and/or increased bone density and strength.

The term "patient" as used herein means a patient in need of being treated or prevented from rheumatoid arthritis, whereas patient means mammals, such as rodents, cows, pigs, dogs, cats, and primates, particularly humans.

The term "pharmaceutical combination" as used herein means a product that results from the mixing or combining of more than one active ingredient and includes both fixed and non-fixed combinations of the active ingredients.

The term "fixed combination" means that the active ingredients, e.g. calcitonin and a co-agent, e.g. a calcium salt and/or a D-type vitamin, are both administered to a patient simultaneously in the form of a single entity or dosage.

The term "non-fixed combination" means that the active ingredients, e.g. calcitonin and a co-agent, e.g. a calcium salt and/or a D-type vitamin, are both administered to a patient as separate entities either simultaneously, concurrently or sequentially with no specific time limits, unless otherwise indicated, wherein such administration provides therapeutically effective levels of the compounds in the body of the patient.

"Oral Unit-Dose Form" refers to physically discrete units suitable for human and animal consumption and packaged individually as is known in the art. It is contemplated for purposes of the present invention that dosage forms of the present invention comprising therapeutically effective amounts of calcitonin and a delivery agent may include one or more unit doses (e.g., tablets, capsules) to achieve the therapeutic effect.

The term "multiple dose" means that pharmaceutical composition according to the invention comprising therapeutically effective amounts of calcitonin and a delivery agent, particularly in form of a oral unit dose will be administered to a human or animal patient in at least two doses in accordance with the dosing interval appropriate for that composition.

The term "single dose" means that the pharmaceutical composition according to the invention comprising therapeutically effective amounts of calcitonin and a delivery agent, particularly in form of a oral unit dose will be administered to a human or animal patient in a single dose.

D type vitamins are a group of fat-soluble prohormones, the two major forms of which are vitamin D2 (or ergocalciferol) and vitamin D3 (or cholecalciferol). The term vitamin D also refers to metabolites and other analogues of these substances. Vitamin D3 is produced in skin exposed to sunlight, specifically ultraviolet B radiation. Vitamin D regulates, with other compounds, the calcium and phosphorus levels in the blood by promoting their absorption from food in the intestines, and by promoting re-absorption of calcium in the kidneys. Vitamin D promotes bone formation and mineralization and inhibits parathyroid hormone secretion from the parathyroid gland. The two major forms most preferred for the invention are vitamin D2 or ergocalciferol, and vitamin D3 or cholecalciferol. The group of D vitamins comprise: Vitamin D1, the molecular compound of ergocalciferol with lumisterol, 1:1, Vitamin D2, ergocalciferol or calciferol (synthesized from ergosterol), Vitamin D3: cholecalciferol (synthesized from 7-dehydrocholesterol in the skin), Vitamin D4, dihydrotachysterol and Vitamin D5, sitocalciferol (synthesized from 7-dehydrositosterol).

### Description

At therapeutic doses, calcitonin has minimal and well-defined side effects. There is a wide therapeutic margin, with only transient and non-life threatening adverse effects with doses many hundred-fold greater than the recommended therapeutic dose. Intranasal and subcutaneous (s.c.) calcitonin administration is occasionally hampered by administration difficulties and local irritation. Some patients are reluctant to administer drugs intranasally or parenterally limiting the potential treatment population. Calcitonin administration is only fully effective when sufficient calcium and vitamin D levels are available in the circulation of the treated subject.

An oral form of calcitonin would improve patient acceptance and facilitate improved patient compliance by providing a more acceptable, alternative delivery route for peptide therapy. Oral application of calcitonin as a combination of salmon calcitonin and 8-(N-2-hydroxy-5-chloro-benzoyl)-amino-caprylic acid (5-CNAC) leads to a marked increase of gastrointestinal (GI) absorption of the sCT in rodents, monkeys and humans. Salmon calcitonin appears rapidly in the plasma following oral administration, with median tₘₐₓ at about 0.25 hr post-dose. Following tₘₐₓ the plasma concentration fell rapidly. No significant accumulation is observed, when comparing area under the curve (AUC) levels after multiple-dose with those after single-dose. Plasma sCT concentrations increased as sCT doses increased. However, with high variability in available data, dose-proportional increase in AUC could not be confirmed. Dosing frequency (q.d. versus b.i.d.) and intensity does not significantly influence sCT PK parameters. Administering calcitonin with food markedly reduces the bioavailability of sCT and preferably oral calcitonin compositions are taken at least 30 minutes, and preferably between 30 and 120 minutes before food intake, which can be breakfast, lunch or dinner.

Pharmacodynamic effects include small decreases in plasma calcium levels, which do not appear to be dose-dependent, and significant suppression of serum CTX-I concentration (a marker of bone resorption), which is dose-related. There are also dose-related decreases in ionized blood calcium, increases in initial (0-1.5 hr) urinary calcium excretion, and decreases in later (9-12 hr) urinary calcium excretion and in serum phosphate.

It would therefore be desirable to improve the pharmacokinetic and pharmacodynamic properties of orally administered calcitonin, to reduce the amount of calcitonin required per dose, and to offset certain metabolic side effects on calcium metabolism by calcitonin administration.

The present invention provides orally effective pharmaceutical compositions comprising a therapeutically effective amount of calcitonin combined with an effective amount of a D type vitamin and calcium. Although the invention discloses orally administered fixed dose combinations of oral calcitonin with vitamin D and/or calcium, the invention also comprises separate administration of calcitonin with vitamin D and/or calcium, either simultaneously or sequentially, in any order or dose regimen.

The present invention also relates a method of treating a subject oral formulations comprising calcitonin and supplementing preferably a D type vitamin and more preferably vitamin D in combination with a calcium salt.

A preferred amount of calcitonin in the composition is from 0.1 to 2.5 mg, more preferably from 0.4 to 1.2 mg, such as 0.6 to 1.2 mg.

Oral dosage forms of pharmaceutical compositions comprising calcitonin and 5CNAC according to the invention are preferably administered at least about 30 minutes, such as at least about 45, 50 or 60 mins, and preferably up to about two hours before the intake of food.

In a preferred embodiment, the oral dosage forms are administered together with a fixed amount of fluid, preferably water, or an aqueous solution. The amount of fluid is preferably at least about 50, 100 or 150 ml, and typically less than about 200 ml.

In one embodiment of the invention, the vitamin D is combined in the oral pharmaceutical dosage form comprising 5CNAC and calcitonin, and the calcium salt is dissolved and administered together with the prescribed amount of water or aqueous solution.

In another embodiment, the vitamin D and the calcium salt are combined in one oral solid dosage form, containing calcitonin and 5CNAC, which is preferably administered with an aqueous solution, in a volume of from about 50 to 200 ml.

When the pharmacologically active agent is salmon calcitonin, the appropriate dosage will, of course, vary depending upon, for example, the host and the nature and severity of the condition being treated. However, in general, satisfactory results will be obtained systemically at daily dosages of from about 0.5 µg/kg to about 10 µg/kg animal body weight, preferably 1 µg/kg to about 6 µg/kg body weight. Expressed in absolute amounts, a preferred amount of calcitonin to be administered is from 0.1 to 2.5 mg/day, more preferably from 0.4 to 1.2 mg/day, such as 0.6 to 1.2 mg/day.

The pharmacologically active agent generally comprises from 0.05 to 70 percent by weight relative to the total weight of the overall pharmaceutical composition, preferably an amount of from 0.01 to 50 percent by weight, more preferably 0.3 to 30 percent by weight relative to the total weight of the overall pharmaceutical composition.

The dose of vitamin D to be combined with calcitonin according to the invention is between 100 and 1000 IU per day (2.5 to 25 micrograms/day), more preferably between 200 and 800 and most preferably between 400 and 800 IU per day, preferably vitamin D2, most preferably vitamin D3 or combinations thereof.

The dose of calcium-salt to be combined with calcitonin, with or without vitamin D, according to this invention, is between about 100 and about 2000 mg of calcium per day, more preferably between 500 and 1500 mg per day and most preferably between 800 and 1000 mg per day.

Any pharmaceutically acceptable source of calcium may be used according to the invention, including food sources rich in calcium, such as milk or cereals. Calcium carbonate is the most common and least expensive calcium supplement. Calcium gluconolactate, optionally in combination with calcium carbonate is preferred for this invention. Calcium carbonate alone can be difficult to digest and causes gas in some people. Taking magnesium with it can help to prevent constipation. Calcium carbonate comprises 40% elemental calcium and 1000 mg will provide 400 mg of free calcium. Calcium citrate may also be used according to the invention. Calcium citrate is more easily absorbed, easier to digest and less likely to cause constipation and gas than calcium carbonate and has a lower risk of contributing to the formation of kidney stones. Calcium citrate comprises about 21% elemental calcium and 1000 mg will provide 210 mg of calcium. Other suitable alternatives comprise, but are not limited to, calcium phosphate, calcium lactate and calcium aspartate.

The delivery agents useful in the present invention are any agents useful for delivering the particular pharmacologically active agent. Suitable delivery agents are any one of the 123 modified amino acids disclosed in aforementioned U.S. Pat. No. 5,866,536 or any one of the 193 modified amino acids described in the aforementioned U.S. Pat. No. 5,773,647 or any combination thereof. The contents of the aforementioned U.S. Pat. Nos. 5,773,647 and 5,866,536 are hereby incorporated by reference in their entirety. In addition, the delivery agent can be the disodium salt of any of the aforementioned modified amino acids as well as ethanol solvates and hydrates thereof. Suitable compounds include compounds of the following formula I 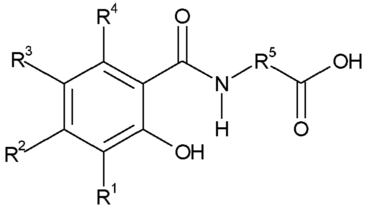 wherein
R¹, R², R³, and R⁴ are independently hydrogen, -OH, -NR⁶R⁷, halogen, C₁-C₄alkyl, or C₁-C₄alkoxy;
R⁵ is a substituted or unsubstituted C₂-C₁₆alkylene, substituted or unsubstituted C₂-C₁₆alkenylene, substituted or unsubstituted C₁-C₁₂alkyl(arylene), or substituted or unsubstituted aryl(C₁-C₁₂alkylene); and R⁶ and R⁷ are independently hydrogen, oxygen, or C₁-C₄ alkyl; and a disodium salt, hydrates and alcohol solvates thereof.

The compounds of formula I as well as their disodium salts and alcohol solvates and hydrates thereof are described in WO 00/059863, along with methods for preparing them.

The disodium salt may be prepared from the ethanol solvate by evaporating or drying the ethanol solvate by methods known in the art to form the anhydrous disodium salt. Drying is generally carried out at a temperature of from about 80 to about 120°C, preferably from about 85 to about 90°C, and most preferably at about 85°C. The drying step is generally performed at a pressure of about 660mm Hg (8.8 kPa) or greater. The anhydrous disodium salt generally contains less than about 5% by weight of ethanol and preferably less than about 2% by weight of ethanol, based on 100% total weight of anhydrous disodium salt.

The disodium salt of the delivery agent can also be prepared by making a slurry of the delivery agent in water and adding two molar equivalents of aqueous sodium hydroxide, sodium alkoxide or the like. Suitable sodium alkoxides include, but are not limited to, sodium methoxide, sodium ethoxide, and combinations thereof.

A still further method of preparing the disodium salt is by reacting the delivery agent with one molar equivalent of sodium hydroxide to yield the disodium salt.

The disodium salt can be isolated as a solid by concentrating the solution containing the disodium salt to a thick paste by vacuum distillation. This paste may be dried in a vacuum oven to obtain the disodium salt of the delivery agent as a solid. The solid can also be isolated by spray drying an aqueous solution of the disodium salt.

The delivery agents may be prepared by methods known in the art, e.g., as mentioned above, by methods described in U.S. Pat. Nos. 5,773,647 and 5,866,536.

The ethanol solvates, as described in the aforementioned WO 00/059863, include, but are not limited to, a molecular or ionic complex of molecules or ions of ethanol solvent with molecules or ions of the disodium salt of the delivery agent. Typically, the ethanol solvate contains about one ethanol molecule or ion for every molecule of disodium salt of the delivery agent.

The ethanol solvate of the disodium salt of the delivery agent can be prepared by dissolving the delivery agent in ethanol. Typically, each gram of delivery agent is dissolved in from about 1 to about 50 ml of ethanol and generally, from about 2 to about 10 ml of ethanol. The delivery agent/ethanol solution is then reacted with a molar excess of a sodium containing salt, such as a monosodium containing salt, relative to delivery agent, i.e. for every mole of delivery agent there is more than one mole of sodium cations, yielding the ethanol solvate. Suitable monosodium salts include, but are not limited to, sodium hydroxide; sodium alkoxides, such as sodium methoxide and sodium ethoxide; and any combination of the foregoing. Preferably, at least about two molar equivalents of the monosodium containing salt are added to the ethanol solution, i.e. for every mole of delivery agent there is at least about two moles of sodium cations. Generally, the reaction is performed at or below the reflux temperature of the mixture, such as at ambient temperature. The ethanol solvate is then recovered by methods known is the art, such as, concentration of the resulting slurry at atmospheric distillation, cooling the concentrated slurry and filtering the solid. The recovered solid can then be vacuum dried to obtain the ethanol solvate.

The hydrates of the disodium salts of the delivery agents may be prepared by drying the ethanol solvate to from an anhydrous disodium salt, as described above, and hydrating the anhydrous disodium salt. Preferably, the monohydrate of the disodium salt is formed. Since the anhydrous disodium salt is very hydroscopic, the hydrate forms upon exposure to atmospheric moisture. Generally, the hydrating step is performed at from about ambient temperature to about 50°C, preferably ambient temperature to about 30°C and in an environment having at least 50% relative humidity. Alternatively, the anhydrous disodium salt may be hydrated with steam.

The preferred delivery agents are N-(5-chlorosalicyloyl)-8-aminocaprylic acid (5-CNAC), N-(10-[2-hydroxybenzoyl]amino)decanoic acid (SNAD), N-(8-[2-hydroxybenzoyl]amino)caprylic acid (SNAC) and their monosodium and disodium salts, ethanol solvates of their sodium salts and the monohydrates of their sodium salts and any combinations thereof. The most preferred delivery agent is the disodium salt of 5-CNAC and the monohydrate thereof.

The pharmaceutical compositions of the present invention typically contain an effective amount of one or more of the delivery agents, i.e. an amount sufficient to deliver the active agent for the desired effect. Generally, the delivery agent is present in an amount of 2.5% to 99.4% by weight, more preferably 15% to 75% by weight, such as at least 25, 30 or 35% but equal to or less than 70 or 60% by weight.

The pharmaceutical compositions of the present invention may be provided as a capsule including a soft-gel capsule, tablet, caplet or other solid oral dosage form, all of which can be prepared by methods well known in the art.

The compositions may additionally comprise additives in amounts customarily employed including, but not limited to, a pH adjuster, a preservative, a flavorant, a taste-masking agent, a fragrance, a humectant, a tonicifier, a colorant, a surfactant, a plasticizer, a lubricant such as magnesium stearate, a flow aid, a compression aid, a solubilizer, an excipient, a diluent such as microcrystalline cellulose, e.g. Avicel PH 102 supplied by FMC corporation, or any combination thereof, with microcrystalline cellulose being particularly preferred. Other additives may include phosphate buffer salts, citric acid, glycols, and other dispersing agents.

Preferably, the solid pharmaceutical compositions of the instant invention include a diluent, such as microcrystalline cellulose (e.g. Avicel), and a lubricant, such as magnesium stearate.

In a particular embodiment, the compositions of the invention may also comprise povidone and/or crospovidone. The crospovidone can be any crospovidone. Crospovidone is a synthetic crosslinked homopolymer of N-vinyl-2-pyrrolidone, also called 1-ethenyl-2-pyrrolidinone, having a molecular weight of 1,000,000 or more. Commercially available crospovidones include Polyplasdone XL, Polyplasdone XL-10, Polyplasdone INF-10 available from ISP, Kollidon CL, available from BASF Corporation. The preferred crospovidone is Polyplasdone XL. Povidone is a synthetic polymer consisting of linear 1-vinyl-2-pyrrolidinone groups having a molecular weight generally between 2,500 and 3,000,000. Commercially available povidones include Kollidon K-30, Kollidon K-90F available from BASF Corporation and Plasdone K-30 and Plasdone K-29/32, available from ISP. As mentioned above, the crospovidones and povidones are commercially available. Alternatively, they may be synthesized by known processes. The crospovidone, povidone or combination thereof is generally present in the compositions in an amount of from 0.5 to 50 percent by weight relative to the total weight of the overall pharmaceutical composition, preferably an amount of from 2 to 25 percent, more preferably 5 to 20 percent by weight relative to the total weight of the pharmaceutical composition.

The solid pharmaceutical compositions of the instant invention can be prepared by conventional methods e.g. by blending a mixture of the active agent or active agents, the delivery agent, the Calcium salt, vitamin D and other ingredients, kneading, and filling into capsules or, instead of filling into capsules, molding followed by further tableting or compression-molding to give tablets. In addition, a solid dispersion may be formed by known methods followed by further processing to form a tablet or capsule.

Preferably, the ingredients in the pharmaceutical compositions of the instant invention are homogeneously or uniformly mixed throughout the solid dosage form.

The compositions of the present invention may be administered to deliver an active agent to any animal in need thereof, including, but not limited to, mammals, such as rodents, cows, pigs, dogs, cats, and primates, particularly humans.

The invention further pertains to a method of preventing or treating disorders responsive to the action of calcitonin in a patient in need thereof comprising administering orally to said patient a therapeutically effective amount of a therapeutic composition this invention, in particular where the calcitonin responsive disorder is a bone disorder. The bone disorder may be a disorder caused by bone resorption including hypercalcemia, osteoporosis, osteolyisis, Paget's disease, disorders characterized by arthritic conditions including Rheumatoid arthritis, inflammation of the joints, swelling of skeletal joints, cartilage erosion, bone erosion, bone fractures, osteoarthritis and bone destruction

The invention further pertains to the combined use of calcitonin, a D-type vitamin and calcium for the manufacture of a medicament for preventing or treating disorders responsive to the action of calcitonin, wherein the calcitonin responsive disorder may a bone resorption disorder, comprising hypercalcemia, osteoporosis, osteolyisis, Paget's disease, disorders characterized by arthritic conditions including rheumatoid arthritis, inflammation of the joints, swelling of skeletal joints, cartilage erosion, bone erosion, bone fractures, osteoarthritis and bone destruction.

The invention also provides a kit of parts comprising:
a) an oral pharmaceutical composition comprising calcitonin with an oral delivery agent selected from the group consisting of N-(5-chlorosalicyloyl)-8-aminocaprylic acid, N-(10-[2-hydroxybenzoyl] aminodecanoic acid or N-(8-[2-hydroxybenzoyl]amino)caprylic acid, or a hydrate, solvate or salt thereof;
b) a composition comprising a D-type vitamin,
c) a composition comprising calcium, and optionally
d) written instructions which instructions provide that said oral pharmaceutical composition may be taken prior to the consumption of food.

Preferably the kit of the invention comprises a composition wherein the calcitonin is about 0.1-2.5 mg of salmon calcitonin, more preferably from 0.6-1.2 mg of salmon calcitonin. In one embodiment it is also preferred that the ratio of the amount of the oral delivery agent, expressed as the corresponding amount of free acid, to the amount of salmon calcitonin is in the range of about 10 to about 250:1 by weight.

The following examples serve to further illustrate the invention but are by no means meant to restrict the scope of the underlying invention.

### Example

A randomized, double-blind, multi-center, placebo-controlled study to evaluate the efficacy and safety of oral salmon calcitonin in the treatment of osteoporosis in postmenopausal women taking calcium and vitamin D is carried out. Subjects are randomized in a blinded fashion to receive either Oral Salmon Calcitonin 0.8 mg per day plus calcium and vitamin D, or placebo plus calcium and vitamin D using a randomization ratio of 1:1. All subjects will participate in the treatment phase for 36 months.

The results assessed are the number of patients with new vertebral fractures and the number of patients with non-vertebral fractures. The study includes adverse events by system organ class and lowest level term, changes in safety laboratory analyses, and number of subjects with antibodies. Subjects receive: 0.8 mg of oral calcitonin with 5-CNAC, plus 800 to 1000 mg of calcium with 400 to 800 IU of vitamin D, the control group a placebo plus 800 to 1000 mg of calcium with 400 to 800 IU of vitamin D. All subjects take one tablet of study medication per day in the evening about 30 to 60 minutes before dinner. The study demonstrates the benefit of oral calcitonin plus calcium and vitamin D compared to calcium and vitamin D alone to reduce the number of subjects with new vertebral and non-vertebral fractures.

The various features and embodiments of the present invention, referred to in individual sections above apply, as appropriate, to other sections, mutatis mutandis. Consequently features specified in one section may be combined with features specified in other sections, as appropriate.

Various modifications and variations of the described methods and products of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in the relevant fields are intended to be within the scope of the following claims

## Claims

1. An oral pharmaceutical composition comprising
(a) an oral delivery agent selected from the group consisting of
(a1) N-(5-chlorosalicyloyl)-8-aminocapric acid (5-CNAC),
(a2) N-(10-[2-hydroxybenzoyl]amino)decanoic acid (SNAD),
(a3) N-(8-[2-hydroxybenzoyl]amino)carprylic acid (SNAC),
and a hydrate, solvate or a salt thereof; and
(b) from 0.1 to 2.5 mg of calcitonin; and
(c1) a D type vitamin selected from the group consisting of Vitamin D1, Vitamin D2, Vitamin D3, Vitamin D4 and Vitamin D5 in an amount of from 200 to 1000 IU; and/or
(c2) a calcium salt in an amount of from 200 to 1000 mg free calcium.

2. An oral pharmaceutical composition according to claim 1 wherein said calcitonin is selected from the group consisting of salmon calcitonin, (Asu 1-7)-eel calcitonin and human calcitonin, and salts thereof.

3. An oral pharmaceutical composition according to any one of the preceding claims wherein said delivery agent is a sodium salt of 5-CNAC.

4. An oral pharmaceutical composition according to any one of the preceding claims wherein said delivery agent is 5-CNAC in free or salt form.

5. An oral pharmaceutical composition according to any one of the preceding claims which is provided in a solid oral dosage form.

6. An oral pharmaceutical composition according to any one of the preceding claims for use in a method of preventing or treating a bone disorder responsive to the action of calcitonin.

7. An oral pharmaceutical composition according to claim 6 wherein said bone disorder is a disorder caused by bone resorption including hypercalcemia, osteoporosis, osteolyisis, Paget's disease, disorders **characterized by** arthritic conditions including Rheumatoid arthritis, inflammation of the joints, swelling of skeletal joints, cartilage erosion, bone erosion, bone fractures, osteoarthritis and bone destruction.

## Patentansprüche

1. Orale pharmazeutische Zubereitung, enthaltend
(a) ein orales Freisetzungsmittel ausgewählt aus der Gruppe, die gebildet wird von
(a1) N-(5-chlorosalicyloyl)-8-aminocapronsäure (5-CNAC)
(a2) N-(10-[2-hydroxy benzoyl]amino)laurinsäure (SNAD)
(a3) N-(8-[2-hydroxy benzoyl]amino)caprylsäure (SNAC) und einem Hydrat, Solvat oder Salz davon, und
(b) von 0.1 bis 2,5 mg Calcitonin ; und
(c1) einem Vitamin vom D-Typ ausgewählt aus der Gruppe, die gebildet wird von Vitamin D1, Vitamin D2, Vitamin D3, Vitamin D4, Vitamin D5 in einer Menge von 200 bis 1.000 IU ; und/oder
(c2) einem Calciumsalz in einer Menge von 200 bis 1.000 mg freiem Calcium.

2. Orale pharmazeutische Zusammensetzung nach Anspruch 1, wobei besagtes Calcitonin ausgewählt ist aus der Gruppe, die gebildet wird von Lachs-Calcitonin, (Asu -7)-eel Calcitonin und humanem Calcitonin sowie Salzen davon.

3. Orale pharmazeutische Zusammensetzung nach einem der vorhergehenden Ansprüche, wobei besagtes Freisetzungsmittel das Natriumsalz des 5-CNAC ist.

4. Orale pharmazeutische Zusammensetzung nach einem der vorhergehenden Ansprüche, wobei besagtes Freisetzungsmittel 5-CNAC in freier Form oder als Salz vorliegt.

5. Orale pharmazeutische Zusammensetzung nach einem der vorhergehenden Ansprüche, wobei diese in fester oraler Dosierform verabreicht wird.

6. Orale pharmazeutische Zusammensetzung nach einem der vorhergehenden Ansprüche zur Verwendung in einem Verfahren zum Schutz oder zur Behandlung von Knochenerkrankungen, die auf die Wirkung von Calcitonin ansprechen.

7. Orale pharmazeutische Zusammensetzung nach Anspruch 6, wobei besagte Knochenerkrankung eine Erkrankung darstellt, die ausgelöst wird durch Knochenresorption einschließlich Hypercalcemie, Osteoporose, Osteolyse, Pagets' Krankheit, Erkrankungen, die ausgezeichnet sind durch arthritische Bedingungen einschließlich Rheumatoide Arthritis, Entzündungen der Gelenke, Schwellungen der Skelettgelenke, Knorpelerosion, Knochenerosion, Knochenbrüche, Osteoarthritis und Knochenzerstörung.

## Revendications

1. Composition pharmaceutique orale comprenant
(a) un agent d'acheminement oral choisi parmi le groupe consistant de
(a1) N-(5-chlorosalicyloyl)-8-acide aminocaproïque (5-CNAC)
(a2) N-(10-[2-hydroxy benzoyl]amino)acide laurique (SNAD)
(a3) N-(8-[2-hydroxy benzoyl]amino)acide caprylique (SNAC) et un hydrate, un solvate ou un sel de celui-ci ; et
(b) de 0,1 à 2,5 mg de la calcitonine ; et
(c1) un type de vitamine D choisi parmi le groupe consistant de la vitamine D1, de la vitamine D2, de la vitamine D3, de la vitamine D4 et de la vitamine D5 d'un montant de 200 à 1000 IU ; et/ou
(c2) un sel de calcium d'un montant de 200 à 1000 mg de calcium libre.

2. Composition pharmaceutique orale selon la revendication 1 dans lequel ladite calcitonine est choisie parmi le groupe consistant de la calcitonine de saumon, (Asu 1-7)-eel calcitonine et de la calcitonine humaine et des sels de celui-ci.

3. Composition pharmaceutique orale selon une des revendications précédentes dans lequel ledit agent d'acheminement est du sel disodique de 5-CNAC.

4. Composition pharmaceutique orale selon une des revendications précédentes dans lequel ledit agent d'acheminement est 5-CNAC sous forme libre ou sous forme de sel.

5. Composition pharmaceutique orale selon une des revendications précédentes qui est mis à disposition sous forme de comprimés oraux.

6. Composition pharmaceutique orale selon une des revendications précédentes pour l'utilisation d'une méthode destinée à prévenir ou à traiter les maladies répondant à l'action de la calcitonine.

7. Composition pharmaceutique orale selon la revendication 6 dans lequel ladite maladie des os est une maladie causée par une résorption osseuse comprenant l'hypercalcémie, l'ostéoporose, l'ostéolyse, la maladie de Paget, les maladies **caractérisées par** des conditions arthritiques comprenant l'arthrite rhumatoïde, l'inflammation des joints, le gonflement des joints squelettiques, l'érosion du cartilage, l'érosion osseuse, les fractures osseuses, l'arthrose et la destruction osseuse.
